# EUROPEAN PATENT APPLICATION

(11) **EP 2 962 639 A1**
(43) Date of publication of application: **06.01.2016**
(21) Application number: 14174930.9
(22) Date of filing: 30.06.2014
(51) Int. Cl.: A61B 6/00, G09G 5/395, H04N 5/77

(54) **A fluoroscopy system for detection and real-time display of fluoroscopy images**

(71) Applicant: Agfa Healthcare, 2640 Mortsel (BE)
(72) Inventor: Vandenberghe, Jef, 2640 Mortsel (BE)
(74) Representative: Verbrugghe, Anne Marie L.

(57) **Abstract**

A fluoroscopy system (1) for processing and real-time display of fluoroscopy images (100) comprising:
- a buffer (13) storing a first image (101) when the buffer (13) is below a threshold (3) as a stored image (120);
- a unit (11) processing the stored image (120) and generating a processed image (110);
- a display (12) displaying processed images (110);
- a memory (14) storing a second image (102) as a stored unprocessed image (130) when the buffer (13) is not below the threshold (3), and storing processed images (110) as stored processed images (140).

## Description

### Field of the Invention

The present invention generally relates to real-time processing and rendering of a sequence of images in a medical imaging environment, e.g. a radiographic environment such as for fluoroscopy applications.

Fluoroscopy, also referred to as dynamic digital imaging, is an imaging technique that uses X-rays to obtain real-time moving images of internal structures of a patient through the use of a fluoroscope. In its simplest form, a fluoroscope consists of an X-ray source and fluorescent panel between which a patient is placed. As the X-rays pass through the patient under study, the X-rays are attenuated by varying amounts as they interact with the different internal structures of the body, thereby casting a shadow of the structures on the fluorescent panel. Images are produced on a display as the un-attenuated X-rays interact with atoms in the panel through the photoelectric effect, giving their energy to the electrons. While much of the energy given to the electrons is dissipated as heat, a fraction of it is given off as visible light, producing the images. Modern fluoroscopes couple the panel to an X-ray image intensifier and CCD video camera allowing images to be recorded and displayed.

### Background of the Invention

US6975752 is a granted patent describing an image detection system for real time acquisition and display of radioscopic images, for use in particular in digital fluoroscopy applications. The imaging system comprises a programmable detector framing node controlling generation of radiation and controlling radioscopic image detection. Fluoroscopy images are received from a selected flat panel detector of a plurality of different flat panel detectors. The detector framing node controls events in real time according to an event instruction sequence and communicates the received fluoroscopy images to a memory. A host processor acts as a unit processing fluoroscopy images. Indeed, an application software running on the host processor can display, archive or process the images. When the host processor is not keeping up with the rate of the incoming image sequence, i.e. that it takes more time to process fluoroscopy images than the flat panel detector needs to capture them, the host processor can ignore one or more fluoroscopy images. The detector framing node fills buffers with images. When a first buffer is full, the detector framing node switches to another buffer and interrupts the host processor. The host processor then empties the first buffer before filling a second buffer up.

In US6975752, the number of images processed by the processing unit per unit of time can be lower that the number of images captured per unit of time and stored in the buffers. In other words, it is possible that the processing unit does not process the images fast enough to keep up with the incoming rate of captured images. This results in the accumulation of images in the buffer of the image detection system, and further results in an increase of the delay between the moment an image is captured and the moment the same image is displayed. This increases the risk that the displayed image is not an image that was captured recently by the flat panel detector. The image detection system described in US6975752 therefore does not guarantee a real-time experience to the user of the system.

It is an objective of the present invention to disclose a system and the related method that overcome the above identified shortcomings of existing tools. More particularly, it is an objective to disclose such system and method for detection and real-time display in a fluoroscopic environment, also referred to as a dynamic digital imaging environment, in order to minimize the delay between the exposure of a patient and the display of the captured images, thereby ensuring a real-time display of the fluoroscopy images in a fast and efficient manner. It is a further objective to disclose such a system that minimizes the exposure time needed to perform a diagnosis, thereby reducing the radiation dose received by the patient.

### Summary of the Invention

According to a first aspect of the present invention, the above defined objectives are realized by a fluoroscopy system for processing and real-time display of fluoroscopy images comprising:
- a detector panel adapted to generate the fluoroscopy images;
- a first processing unit adapted to process the fluoroscopy images and to generate processed fluoroscopy images;
- a first display adapted to display the processed fluoroscopy images in a real-time viewing mode;
CHARACTERIZED IN THAT the fluoroscopy system further comprises:
- a buffer adapted to store a first fluoroscopy image of the fluoroscopy images when a buffer filling of the buffer is below a predetermined threshold as a stored fluoroscopy image; and in that
- the first processing unit is further adapted to process the stored fluoroscopy image and to generate a processed fluoroscopy image;
and in that the fluoroscopy system further comprises:
- a memory adapted to store a second fluoroscopy image of the fluoroscopy images as a stored unprocessed fluoroscopy image when the buffer filling of the buffer is not below the predetermined threshold, and to store the processed fluoroscopy image as a stored processed fluoroscopy image.

The fluoroscopy images according to the present invention may for example be images of a specific local and narrow area of the body of the patient under study, for example a finger, an arm, a shoulder, etc. Fluoroscopy images according to the present invention may also capture a larger area of the body of the patient, for example when a radiographic positive contrast agent such as silver, bismuth, caesium, or any iodine-based agent is injected in the patient, or when a negative contrast agent such as carbon dioxide is injected in the patient. The first display according to the present invention may for example be a screen in the examination room, a screen of a desktop or personal computer, a CCD video camera cell coupled to an X-ray image intensifier, or the screen of a laptop, a tablet or smartphone. The memory according to the present invention may for example comprise one or more hard disk or volatile memory on which captured fluoroscopy images may be stored and may therefore be accessible after examination of the patient under study.

According to the present invention, a fluoroscopy image is stored in a buffer, as long as the buffer filling is below a predetermined threshold, before being processed by a first processing unit and displayed by a first display. The processed fluoroscopy image according to the present invention is stored in the memory as a processed fluoroscopy image. The buffer filling is an indication of how full the buffer is. In other words, the buffer filling is an indication on how many fluoroscopy images are stored in the buffer. As long as the buffer filling of the buffer is not below a predetermined threshold, the system according to the invention skips fluoroscopic images and stores them in the memory as unprocessed fluoroscopy images. As soon as the buffer filling of the buffer is below the predetermined threshold, the next fluoroscopy image from the sequence of fluoroscopy images is stored in the buffer, before being retrieved and processed by the first processing unit and being stored in the memory as a processed fluoroscopy image. This way, there is no accumulation of fluoroscopy images in the buffer. The first processing unit according to the present invention processes the fluoroscopy images fast enough to keep up with the incoming rate of fluoroscopy images made available in the buffer. This minimizes the delay between the moment a fluoroscopy image is received, is processed and is displayed on the first display. This provides the user of the system according to the present invention with a real-time experience, as what is displayed on the first display is a recent fluoroscopy image, i.e. as the display is in sync with the capturing of fluoroscopy images. This makes diagnosis reliable and fast. The radiation dose received by the patient under study may therefore be reduced, which also reduces the risk for the health of the patient generated by the fluoroscopy study.

According to the present invention, processed fluoroscopy images are stored in the memory. Also, skipped fluoroscopy images, i.e. unprocessed by the first processing unit, are stored as unprocessed fluoroscopy images in the memory according to the invention. In a preferred embodiment, all the images that are captured are stored in the memory according to the present invention. This way, when the capturing of fluoroscopy images is finished, all the received fluoroscopy images, i.e. processed and unprocessed fluoroscopy images, remain stored in the system according to the invention. It is then be possible to retrieve these fluoroscopy images offline, without requiring a further examination of the patient. This reduces the radiation dose received by the patient and reduces the risk for his health.

According to an optional embodiment, the fluoroscopy system further comprises a detector panel adapted to generate the fluoroscopy images.

In accordance with the present invention, the detector panel may for example be a photographic film, a photo-multiplier tube, a scintillator detector, or an amorphous silicon based photosensitive cell. Fluoroscopy images can also be captured and generated by a flat-panel detector with an increased sensitivity to X-rays or an image intensifier. In other words, fluoroscopy images can be received by the fluoroscopy system from any existent acquisition device.

According to an optional embodiment, the buffer is further adapted to store the first fluoroscopy image when the buffer is empty, and the memory is adapted to store the second fluoroscopy image when the buffer is not empty.

In a preferred embodiment of the invention, a fluoroscopic image captured always enters an empty queue in the buffer. This means that the processing of the previous fluoroscopy image stored in the buffer as a stored fluoroscopy image should be started when a new fluoroscopy image arrives. The predetermined threshold in such embodiment of the invention consequently is zero. This ensures that the first processing unit keeps up with the input rate of captured fluoroscopy images. Skipped fluoroscopy images, i.e. unprocessed by the first processing unit, are stored as unprocessed fluoroscopy images in the memory. This way, when the capturing of fluoroscopy images is finished, unprocessed fluoroscopy images may remain stored in the system according to the invention, and therefore be accessible offline.

According to an optional embodiment, the memory is further adapted to additionally store the first fluoroscopy image.

This way, the memory is adapted to store the first fluoroscopy image. In other words, the first fluoroscopy image is also stored in the memory and is independently and in parallel processed by the first processing unit. This way, when the capturing of fluoroscopy images is finished, all the captured fluoroscopy images, i.e. processed and unprocessed fluoroscopy images, remain persistently stored in the memory, for example on a hard disk and/or a volatile memory. It is then for example possible to retrieve these fluoroscopy images offline, without requiring a further examination of the patient, and to process again the complete sequence of fluoroscopy images according to a different algorithm or to different processing parameters.

According to an optional embodiment, the fluoroscopy system according to the present invention further comprises:
- a second processing unit adapted to retrieve and process the stored unprocessed fluoroscopy image thereby obtaining an offline processed fluoroscopy image;
and the memory according to the present invention is further adapted to store the offline processed fluoroscopy image as a stored offline processed fluoroscopy image.

Thus, optionally, a second processing unit may be adapted to retrieve unprocessed fluoroscopy images from the memory and to process them offline, i.e. when the detector panel is ready with capturing new fluoroscopy images. This generates offline processed fluoroscopy images that may be stored in the memory as stored offline processed fluoroscopy images. This way, captured fluoroscopy images that were skipped for real-time processing because the buffer filling of the buffer was not below a predetermined threshold, may be recovered and processed offline. A full sequence of processed fluoroscopy images and offline processed fluoroscopy images may therefore be reconstructed and displayed offline.

According to an optional embodiment, the processed fluoroscopy image comprises a processing status identifier, and the second processing unit is further configured to identify the stored unprocessed fluoroscopy image when the processing status identifier is not present or has a different value.

This way, a stored processed fluoroscopy image is identified by a processing status identifier. When a fluoroscopy image stored in the memory does not comprise a processing status identifier, or when the processing status identifier is set to a specific value, the second processing unit is able to identify it in the memory as a stored unprocessed fluoroscopy image, amongst all the fluoroscopy images that are stored in the memory. In other words, the second processing unit is able to make the distinction between stored processed fluoroscopy images and stored unprocessed fluoroscopy images. This processing status identifier makes it easier for the second processing unit to identify the stored fluoroscopy images that need processing, and therefore makes the processing of the stored unprocessed fluoroscopy images easier and faster.

According to an optional embodiment, the second processing unit according to the present invention corresponds to the first processing unit.

Indeed, preferably, the first and second processing units are identical. This reduces the complexity and the cost of the system according to the present invention.

According to an optional embodiment, the fluoroscopy system according to the present invention further comprises a second display adapted to display stored processed fluoroscopy images comprising the stored offline processed fluoroscopy image and the stored processed fluoroscopy image from the memory in an offline viewing mode.

Thus, a second display may display a sequence of fluoroscopy images comprising stored offline processed fluoroscopy images and stored processed fluoroscopy images from the memory. This full processed sequence of fluoroscopy images may be used for performing a diagnosis or for playback.

According to an optional embodiment, the first display according to the present invention corresponds to the second display.

Indeed, preferably, also the first and second displays are identical. This further reduces the complexity and the cost of the system according to the present invention.

According to an optional embodiment, the predetermined threshold according to the present invention is calculated from one or more of the following parameters:
- a speed at which fluoroscopy images are processed by the first processing unit; and
- a maximum delay between the capture of the fluoroscopy images by the detector panel and the display of the processed fluoroscopy images.

In accordance with the present invention, the decision of the system to skip a fluoroscopy image during the real-time viewing mode is based on a predetermined threshold indicative for a maximum acceptable fill level of the buffer. The predetermined threshold for instance may be calculated as the product of the speed at which fluoroscopy images are processed by the first processing unit and the maximum acceptable delay between the moment a fluoroscopy image is captured and the moment that it is displayed after processing. Indeed, there may exist a value of the delay considered as acceptable by a user of the system according to the present invention while still providing him with real-time experience. This way, the number of fluoroscopy images that must be skipped and stored in the memory is calculated depending on the speed of the first processing unit and the delay. For example, a delay of 5 milliseconds between the capturing and the display of a fluoroscopy image may be acceptable for a user of the system according to the present invention to preserve a real-time viewing mode. The first processing unit may process 2 images per millisecond. Ten fluoroscopy images may therefore be stored in the buffer to become real-time processed while subsequent fluoroscopy images will be stored in the memory as unprocessed fluoroscopy images. This brings flexibility to the system according to the present invention, as the acceptable delay may vary from one fluoroscopy study to another.

According to an optional embodiment, the memory further comprises one or more hard disk or other volatile memory.

Thus, fluoroscopy images may be stored in one or more hard disk or volatile memory of the memory. This way, processed and unprocessed fluoroscopy images may be retrieved from the system according to the present invention, and accessed offline, even independently from the system.

According to an optional embodiment, the buffer is further adapted to store a first fluoroscopy image of the stored images when the buffer filling of the buffer is below a predetermined threshold as a new stored fluoroscopy image; and
- the processing unit is further adapted to process the new stored offline fluoroscopy image and to generate a new processed fluoroscopy image; and
- the memory is further adapted to store a second fluoroscopy image of the stored fluoroscopy images as a new stored unprocessed fluoroscopy image when the buffer filling of the buffer is not below the predetermined threshold, and to store the new processed fluoroscopy image as a new stored processed fluoroscopy image.

In an advanced embodiment of the invention, a full sequence of stored images are processed again by the first and second processing units. The full sequence of stored images may for instance be processed according to a different algorithm or different parameters. This brings flexibility to the system according to the present invention as the full sequence of stored images may be displayed according to different parameters this way. This may also improve the relevance of the fluoroscopy study, as a user of the system according to the present invention may choose to modify the contrast, the brightness, etc. of the fluoroscopy images in order to improve his diagnosis or to make the analysis of the fluoroscopy images easier.

According to an optional embodiment, the stored images comprise one or more of the following:
- the stored unprocessed fluoroscopy image;
- the stored processed fluoroscopy image; and
- the stored offline processed fluoroscopy image.

Thus, the stored images may comprise images that are unprocessed but stored in the memory of the system, images that are processed by the first processing unit and stored in the memory of the system, and images that are processed offline and stored in the memory of the system. This saves time to a user of the system according to the present invention. For instance, it may be more relevant for the system according to the present invention to process the fluoroscopy images according to different parameters. When the capturing is finished, instead of starting with the processing of stored unprocessed fluoroscopy images according to the same parameters as the ones used to generate the processed fluoroscopy images, the system according to the present invention may modify the processing parameters of the first and second processing units in order to modify the process and eventually the display of the full sequence of newly processed fluoroscopy images.

According to a second aspect of the invention, there is provided a method for detection and real-time display of fluoroscopy images, the method comprising the steps of:
- capturing the fluoroscopy images;
- processing the fluoroscopy images and generating processed fluoroscopy images;
- displaying the processed fluoroscopy images in a real-time viewing mode;
- storing a first fluoroscopy image of the fluoroscopy images in a buffer when the buffer filling of the buffer is below a predetermined threshold as a stored fluoroscopy image;
- processing the stored fluoroscopy image and thereby generating a processed fluoroscopy image;
- storing a second fluoroscopy image of the fluoroscopy images as a stored unprocessed fluoroscopy image when the buffer filling of the buffer is not below the predetermined threshold, and storing the processed fluoroscopy image as a stored processed fluoroscopy image.

In accordance with the present invention, fluoroscopy images are captured. A fluoroscopy image is stored in a buffer as long as the buffer filing is below a predetermined threshold, before being processed and displayed. The processed fluoroscopy image according to the present invention are stored as a processed fluoroscopy image. As long as the buffer filling of the buffer according to the invention is not below a predetermined threshold, fluoroscopy images are skipped and stored as unprocessed fluoroscopy images. As soon as the buffer filling of the buffer according to the present invention is below the predetermined threshold, the next fluoroscopy image from the sequence of fluoroscopy images is stored in the buffer, before being retrieved and processed and being stored as a processed fluoroscopy image. This way, there is no accumulation of fluoroscopy images in the buffer. The fluoroscopy images are processed fast enough to keep up with the incoming rate of fluoroscopy images made available in the buffer. This minimizes the delay between the capturing of a fluoroscopy image, its processing and its display. This ensures a real-time experience, as what is displayed is a recent fluoroscopy image, i.e. as the display is in sync with the capturing of fluoroscopy images. This makes diagnosis reliable and fast. Indeed, the radiation dose received by the patient under study may be reduced, which also reduces the risk for the health of the patient generated by the fluoroscopy study.

The current invention in addition also relates to a computer program comprising software code adapted to perform the method according to the present invention.

The current invention further relates to a computer readable storage medium comprising the computer program according to the present invention.

### Brief Description of the Drawings

Fig. 1 schematically illustrates an embodiment of a system for detection and real-time display of fluoroscopy images.
Fig. 2 schematically illustrates an embodiment of a system for detection and real-time display of fluoroscopy images, where skipped fluoroscopy images are processed offline.
Fig. 3 schematically illustrates an embodiment of a system for detection and real-time display of fluoroscopy images, where offline processed fluoroscopy images and stored fluoroscopy images are displayed.
Fig. 4 schematically illustrates an embodiment of a system for detection and real-time display of fluoroscopy images, where stored images are retrieved from the memory to be processed.
Fig. 5 schematically illustrates a suitable computing system for hosting the system of Fig. 1.

### Detailed Description of Embodiment(s)

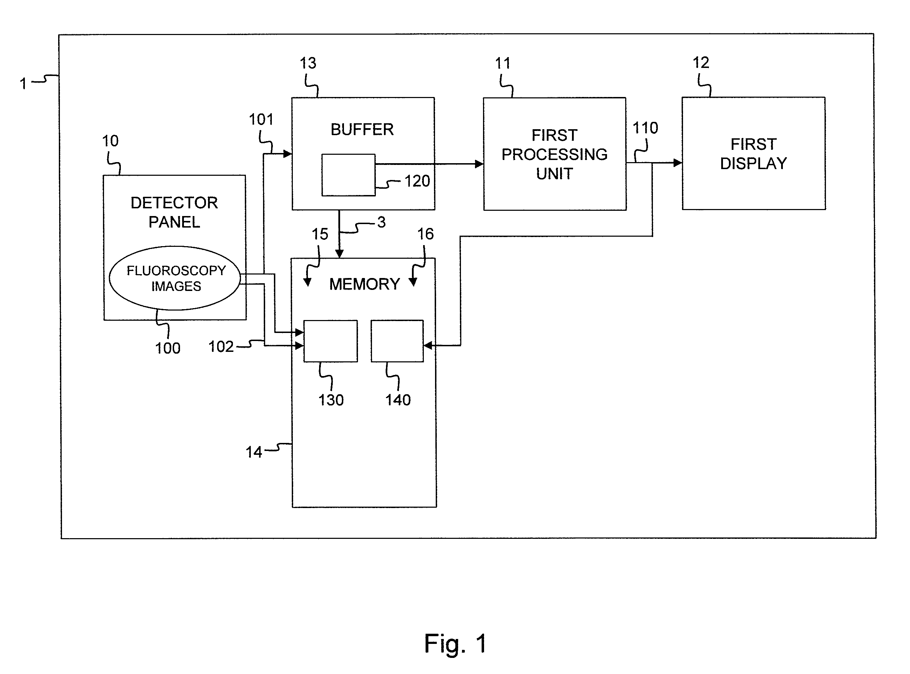

According to an embodiment shown in Fig. 1, a system 1 comprises a detector panel 10, a first processing unit 11, a first display 12, a buffer 13 and a memory 14. A sequence of frames is captured by the detector panel 10, thereby generating a sequence of fluoroscopy images 100. According to an alternative embodiment, the sequence of fluoroscopy images 100 can be received from an acquisition device. As visible in Fig. 1, a first fluoroscopy image 101 of the fluoroscopy images 100 is stored in the buffer 13 when the buffer filling of the buffer 13 is below a predetermined threshold 3. The fluoroscopy image 101 is then stored as a stored fluoroscopy image 120. The stored fluoroscopy image 120 is retrieved from the buffer 13 by the first processing unit 11. The first processing unit 11 processes the stored fluoroscopy image 120, thereby generating a processed fluoroscopy image 110. This processed fluoroscopy image 110 is sent to a first display 12 so that a user of the system 1 can see the fluoroscopy image 101 that was captured by the detector panel 10. The first display 12 is adapted to display the processed fluoroscopy images 110 in real-time viewing mode. The processed fluoroscopy image 110 is also stored in the memory 14 as a stored processed fluoroscopy image 140. Optionally, the stored processed fluoroscopy image 140 can comprise a processing status identifier. As shown in Fig. 1, the first fluoroscopy image 101 can also preferably be stored in the memory 14, to be for example persistently stored on a hard disk 15 or on a volatile memory 16 of the memory 14. This is done independently from and in parallel with the processing of the first fluoroscopy image 101 by the first processing unit 11. When the buffer filling of the buffer 13 is not below a predetermined threshold 3, it is not possible to store a fluoroscopy image from the fluoroscopy images 100 in the buffer 13. A second fluoroscopy image 102 from the fluoroscopy images 100 arriving at a point in time when the buffer fill level exceeds the threshold is then only stored in the memory 14 instead of being also stored in the buffer 13. In other words, the system 1 skips the second fluoroscopy image 102 from the sequence of fluoroscopy images 100 captured by the detector panel 10 for real-time processing when the buffer is filled up to a level above the threshold. The second fluoroscopy image is stored as a stored unprocessed fluoroscopy image 130. As long as the buffer filling of the buffer 13 is not below the predetermined threshold 3, the system 1 keeps skipping fluoroscopic images 100 and storing them in the memory 14 as stored unprocessed fluoroscopy images 130. As soon as the buffer filling of the buffer 13 is below the predetermined threshold, the next fluoroscopy image from the sequence of fluoroscopy images 100 is stored in the buffer 13, and the stored unprocessed fluoroscopy images 130 as well as the processed fluoroscopy images 140 remain stored in the memory 14. According to an alternative embodiment, the stored processed fluoroscopy images 140 and the stored unprocessed fluoroscopy images 130 can both comprise a processing states identifier. The value of the processing status identifier associated with the stored processed fluoroscopy images 140 is different from the value of the processing status identifier associated with the stored unprocessed fluoroscopy images 130. For example, a value of 0 for the processing status identifier can be associated with stored unprocessed fluoroscopy images 130 and a value of 1 for the processing status identifier can be associated with stored processed fluoroscopy images 140. According to a further alternative embodiment, a value of 1 for the processing status identifier can be associated with stored unprocessed fluoroscopy images 130 and a value of 0 for the processing status identifier can be associated with stored processed fluoroscopy images 140. As visible in Fig. 1, the memory 14 comprises one or more hard disk 15 and/or one or more volatile memory 16. Stored unprocessed fluoroscopy images 130 and/or stored processed fluoroscopy images 140 can be stored on one or more hard disk 15 and/or volatile memory 16 and therefore be accessed even when the acquisition by the detector panel 10 is finished and without requiring the presence of the patient under study.

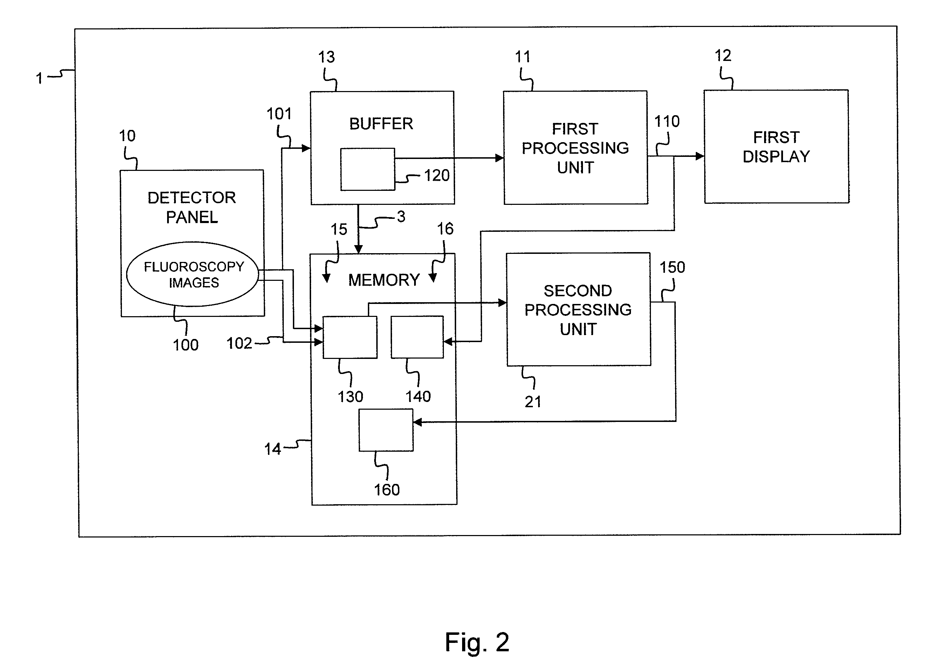

According to an embodiment shown in Fig. 2, a system 1 comprises a detector panel 10, a first processing unit 11, a first display 12, a buffer 13, a memory 14 and a second processing unit 21. Components having identical reference numbers to components in Fig. 1 perform the same function. Optionally, stored processed fluoroscopy images 140 can be identified by the second processing unit 21 when a processing status identifier is present. Optionally, stored unprocessed fluoroscopy images 130 can be identified by the second processing unit 21 when no processing status identifier is present. Stored unprocessed fluoroscopy images 130 are retrieved from the memory 14 by the second processing unit 21 and processed by the second processing unit 21. This process is performed offline, i.e. when the capturing of the sequence of fluoroscopy images 100 by the detector panel 10 is finished. The system 1 is able to process fluoroscopy images 100 that have been captured by the detector panel 10, but that have been skipped from processing by the first processing unit 11 in order to ensure the system 1 was continuously operating in real-time viewing mode. The skipped images of the fluoroscopy images 100 are processed by the second processing unit 21 as offline processed fluoroscopy images 150. The offline processed fluoroscopy images 150 are stored in the memory 14 as stored offline processed fluoroscopy images 160. The memory 14 comprises one or more hard disk 15 and/or one or more volatile memory 16. Stored offline processed fluoroscopy images 160 can be stored on one or more hard disk 15 and/or volatile memory 16 and therefore be accessed even when the acquisition by the detector panel 10 is finished and without requiring the presence of the patient under study.

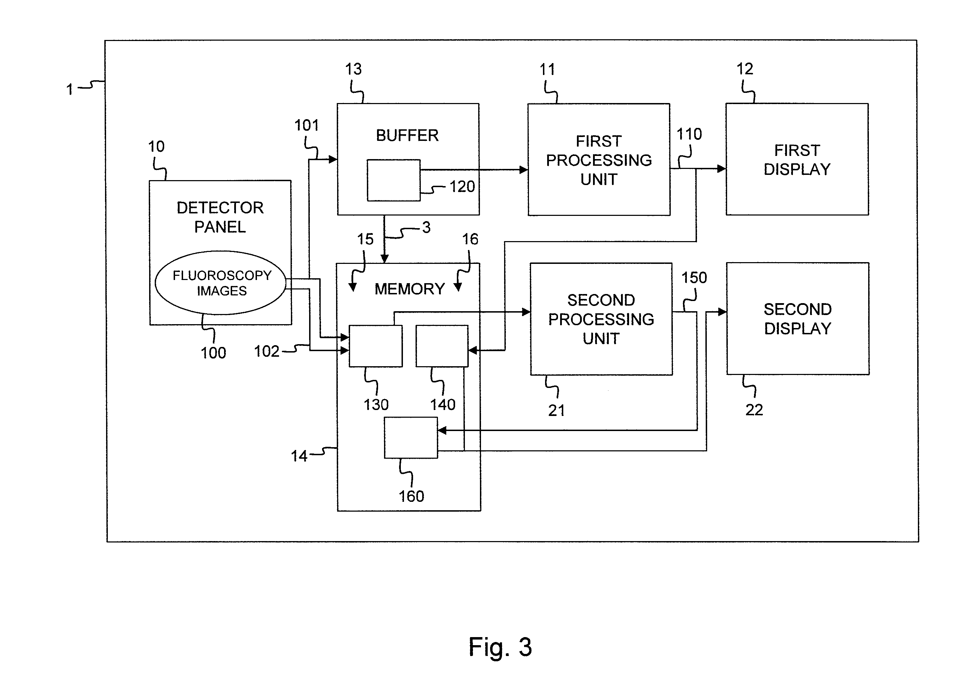

According to an embodiment shown in Fig. 3, a system 1 comprises a detector panel 10, a first processing unit 11, a first display 12, a buffer 13, a memory 14, a second processing unit 21, and a second display 22. Components having identical reference numbers to components in Fig. 1 and 2 perform the same function. As an offline process, stored offline processed fluoroscopy images 160 stored in the memory 14, and stored processed fluoroscopy images 140 stored in the memory 14 are retrieved from the memory 14 and displayed in the second display 22. The system 1 is therefore able to display on the second display 22 the full sequence of fluoroscopy images captured by the detector panel 10. Fluoroscopy images of this sequence have been processed by the first processing unit 11 in real-time mode, i.e. the stored processed fluoroscopy images 140, and other fluoroscopy images of this sequence have been processed by the second processing unit 21 offline, i.e. the stored offline processed fluoroscopy images 160.

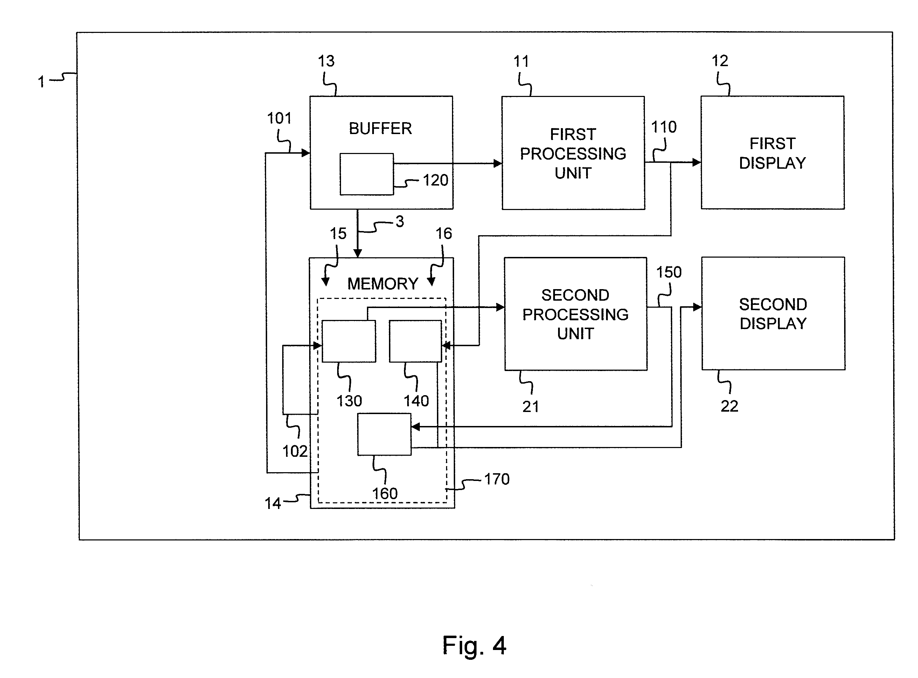

According to an embodiment shown in Fig. 4, a system 1 comprises a first processing unit 11, a first display 12, a buffer 13, a memory 14, a second processing unit 21 and a second display 22. Components having identical reference numbers to components in Fig. 1 to 3 perform the same function. The memory 14 comprises one or more hard disk 15 and/or one or more volatile memory 16. Stored images 170 in the memory 14 comprise stored unprocessed fluoroscopy images 130, stored processed fluoroscopy images 140 and stored offline processed fluoroscopy images 160. Stored images 170 can be stored in one or more hard disk 15 or one or more volatile memory 16 of the memory 14. A first fluoroscopy image 101 of the stored images 170 is stored in the buffer 13 when the buffer filling of the buffer 13 is below a predetermined threshold 3. The fluoroscopy image 101 is then stored as a stored fluoroscopy image 120. The stored fluoroscopy image 120 is retrieved from the buffer 13 by the first processing unit 11. The first processing unit 11 processes the stored fluoroscopy image 120, thereby generating a processed fluoroscopy image 110. This processed fluoroscopy image 110 is sent to a first display 12 so that a user of the system 1 can see the fluoroscopy image 101 that was captured by the detector panel 10. The first display 12 is adapted to display the processed fluoroscopy images 110 in real-time viewing mode. The processed fluoroscopy image 110 is also stored in the memory 14 as a stored processed fluoroscopy image 140. When the buffer filling of the buffer 13 is not below a predetermined threshold 3, it is not possible to store a fluoroscopy image from the fluoroscopy images 100 in the buffer 13. A second fluoroscopy image 102 from the stored images 170 is then only stored in the memory 14 instead of also being stored in the buffer 13. The system 1 thereby skips the second fluoroscopy image 102 from the sequence of stored images 170 from the memory 14 when the buffer filling of the buffer is not below the predetermined threshold. The second fluoroscopy image is stored as a stored unprocessed fluoroscopy image 130. As long as the buffer filling of the buffer 13 is not below the predetermined threshold 3, the system 1 keeps skipping stored images 170 and storing them in the memory 14 as stored unprocessed fluoroscopy images 130. As soon as the buffer filling of the buffer 13 is below the predetermined threshold, the next fluoroscopy image from the sequence of stored images 170 is stored in the buffer 13, and the stored unprocessed fluoroscopy images 130 as well as the processed fluoroscopy images 140 remain stored in the memory 14. Stored unprocessed fluoroscopy images 130 and/or stored processed fluoroscopy images 140 can be stored on one or more hard disk 15 and/or volatile memory 16 and therefore be accessed even when the acquisition by the detector panel 10 is finished and without requiring the presence of the patient under study.

It is clear that in the embodiments described in Fig. 1 to 4, the memory 14 preferentially stores fluoroscopy images independently from and in parallel with the processing of the fluoroscopy images performed by the processing units 11;21, as then, when the capturing of fluoroscopy images is finished, all the captured fluoroscopy images remain stored in the memory 14. It is then be possible to retrieve these fluoroscopy images to process them and to build a new sequence of processed fluoroscopy images. Additionally, the memory 14 is then further able to link processed and unprocessed fluoroscopy images together in order to reconstruct the original sequence of captured fluoroscopy images, and is able to identify which stored fluoroscopy images from the memory need processing.

It is clear that in the embodiments described in Fig. 1 to 4, fluoroscopy images can be easily stored and retrieved from the memory 14, from the hard disk 15 of the memory 14, and/or from the volatile memory 16 of the memory 14.

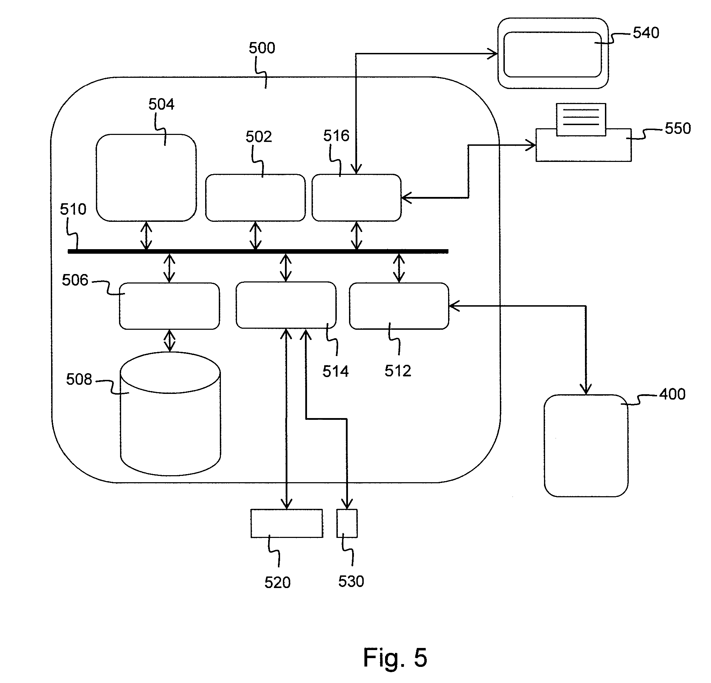

Fig. 5 shows a suitable computing system 500 for hosting the system 1 of Fig. 1 to 4. Computing system 500 may in general be formed as a suitable general purpose computer and comprise a bus 510, a processor 502, a local memory 504, one or more optional input interfaces 514, one or more optional output interfaces 516, a communication interface 512, a storage element interface 506 and one or more storage elements 508. Bus 510 may comprise one or more conductors that permit communication among the components of the computing system. Processor 502 may include any type of conventional processor or microprocessor that interprets and executes programming instructions. Local memory 504 may include a random access memory (RAM) or another type of dynamic storage device that stores information and instructions for execution by processor 502 and/or a read only memory (ROM) or another type of static storage device that stores static information and instructions for use by processor 502. Input interface 514 may comprise one or more conventional mechanisms that permit an operator to input information to the computing device 500, such as a keyboard 520, a mouse 530, a pen, voice recognition and/or biometric mechanisms, etc. Output interface 516 may comprise one or more conventional mechanisms that output information to the operator, such as a display 540, a printer 550, a speaker, etc. Communication interface 512 may comprise any transceiver-like mechanism such as for example two 1 Gb Ethernet interfaces that enables computing system 500 to communicate with other devices and/or systems, for example mechanisms for communicating with one or more other computing systems 400. The communication interface 512 of computing system 500 may be connected to such another computing system by means of a local area network (LAN) or a wide area network (WAN, such as for example the internet, in which case the other computing system 400 may for example comprise a suitable web server. Storage element interface 506 may comprise a storage interface such as for example a Serial Advanced Technology Attachment (SATA) interface or a Small Computer System Interface (SCSI) for connecting bus 510 to one or more storage elements 508, such as one or more local disks, for example 1TB SATA disk drives, and control the reading and writing of data to and/or from these storage elements 508. Although the storage elements 508 above is described as a local disk, in general any other suitable computer-readable media such as a removable magnetic disk, optical storage media such as a CD or DVD, -ROM disk, solid state drives, flash memory cards, ... could be used.

The first processing unit 11 and the second processing unit 21 of the system 1 can be implemented as programming instructions stored in local memory 504 of the computing system 500 for execution by its processor 502. Alternatively the system 1 could be stored on the storage element 508 or be accessible from another computing system 400 through the communication interface 512.

In accordance with the present invention, engines may be realized in software, or hardware or as a combination of thereof.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A fluoroscopy system (1) for processing and real-time display of fluoroscopy images (100) comprising:
- a first processing unit (11) adapted to receive and process fluoroscopy images (100) and to generate processed fluoroscopy images (110);
- a first display (12) adapted to display said processed fluoroscopy images (110) in a real-time viewing mode;
**CHARACTERIZED IN THAT** said fluoroscopy system (1) further comprises:
- a buffer (13) adapted to store a first fluoroscopy image (101) of said fluoroscopy images (100) when a buffer filling of said buffer (13) is below a predetermined threshold (3) as a stored fluoroscopy image (120); and **in that**
- said first processing unit (11) is further adapted to process said stored fluoroscopy image (120) and to generate a processed fluoroscopy image (110);
and **in that** said fluoroscopy system (1) further comprises:
- a memory (14) adapted to store a second fluoroscopy image (102) of said fluoroscopy images (100) as a stored unprocessed fluoroscopy image (130) when said buffer filling of said buffer (13) is not below said predetermined threshold (3), and to store said processed fluoroscopy image (110) as a stored processed fluoroscopy image (140).

2. A fluoroscopy system (1) according to claim 1, wherein said fluoroscopy system (1) further comprises a detector panel (10) adapted to generate said fluoroscopy images (100).

3. A fluoroscopy system (1) according to claim 1, wherein said buffer (13) is adapted to store said first fluoroscopy image (101) when said buffer filling of said buffer (13) is empty; and wherein said memory (14) is adapted to store said second fluoroscopy image (102) when said buffer filling of said buffer (13) is not empty.

4. A fluoroscopy system (1) according to claim 1, wherein said memory (14) is further adapted to additionally store said first fluoroscopy image (101).

5. A fluoroscopy system (1) according to claim 1 or 3 comprising:
- a second processing unit (21) adapted to retrieve and process said stored unprocessed fluoroscopy image (130) thereby obtaining an offline processed fluoroscopy image (150); and
wherein said memory (14) is further adapted to store said offline processed fluoroscopy image (150) as a stored offline processed fluoroscopy image (160).

6. A fluoroscopy system (1) according to claim 1, wherein:
- said processed fluoroscopy image (140) comprises a processing status identifier; and
- said second processing unit (21) is further configured to identify said stored unprocessed fluoroscopy image (130) when said processing status identifier is not present or has a different value.

7. A fluoroscopy system (1) according to claim 5, wherein said second processing unit (21) corresponds to said first processing unit (11).

8. A fluoroscopy system (1) according to claim 5 further comprising:
- a second display (22) adapted to display said stored offline processed fluoroscopy image (160) and said stored processed fluoroscopy image (140) from said memory (14) in an offline viewing mode.

9. A fluoroscopy system (1) according to claim 8, wherein said first display (12) corresponds to said second display (22).

10. A fluoroscopy system (1) according to claim 1, wherein said predetermined threshold (3) is calculated from one or more of the following parameters:
- a speed at which fluoroscopy images (100) are processed by said first processing unit (11); and
- a maximum delay (5) between the capture of said fluoroscopy images (100) by said detector panel (10) and the display of said processed fluoroscopy images (110).

11. A fluoroscopy system (1) according to claims 1 and 3 and 4, wherein:
- said buffer (13) is further adapted to store a first fluoroscopy image (101) of stored images (170) when said buffer filling of said buffer (13) is below a predetermined threshold (3) as a new stored fluoroscopy image (121);
- said processing unit (11) is further adapted to process said new stored fluoroscopy image (121) and to generate a new processed fluoroscopy image (131); and
- said memory (14) is further adapted to store a second fluoroscopy image (102) of said stored images (170) as a new stored unprocessed fluoroscopy image (141) when said buffer filling of said buffer (13) is not below said predetermined threshold (3), and to store said new processed fluoroscopy image (131) as a new stored processed fluoroscopy image (151).

12. A fluoroscopy system (1) according to claims 1 and 5 and 11 wherein said stored images (170) comprise one or more of the following:
- said stored unprocessed fluoroscopy image (130);
- said stored processed fluoroscopy image (140); and
- said stored offline processed fluoroscopy image (160).

13. A method for processing and real-time display of fluoroscopy images (100), said method comprising the steps of:
- receiving fluoroscopy images (100);
- processing said fluoroscopy images (100) and generating processed fluoroscopy images (110);
- displaying said processed fluoroscopy images (110) in a real-time viewing mode;
- storing a first fluoroscopy image (101) of said fluoroscopy images (100) in a buffer (13) when said buffer filling of said buffer (13) is below a predetermined threshold (3) as a stored fluoroscopy image (120);
- processing said stored fluoroscopy image (120) and thereby generating a processed fluoroscopy image (110);
- storing a second fluoroscopy image (102) of said fluoroscopy images (100) as a stored unprocessed fluoroscopy image (130) when said buffer filling of said buffer (13) is not below said predetermined threshold (3), and storing said processed fluoroscopy image (110) as a stored processed fluoroscopy image (140).

14. A computer program comprising software code adapted to perform the method of claim 13.

15. A computer readable storage medium comprising computer-executable instructions which, when executed by a computing system, perform the method of claim 13.
